# EUROPÄISCHE PATENTSCHRIFT

(11) **EP 2 208 780 B1**
(45) Veröffentlichungstag und Bekanntmachung des Hinweises auf die Patenterteilung: **01.11.2017**
(21) Anmeldenummer: 09000701.4
(22) Anmeldetag: 20.01.2009
(51) Int. Cl.: C12M 1/42, C12M 3/00

(54) **Verfahren und Vorrichtung zur elektrischen Behandlung mehrerer Behältnisse**
Method and device for electric processing of multiple containers
Procédé et dispositif destinés au traitement électrique de plusieurs récipients

(43) Veröffentlichungstag der Anmeldung: 21.07.2010
(73) Patentinhaber: Lonza Cologne GmbH, 50829 Köln (DE)
(72) Erfinder: Altrogge, Ludger, 53894 Mechernich (DE); Gleißner, Timo, 53879 Euskirchen (DE); Heinze, Andreas, 50739 Köln (DE); Müller-Hartmann, Herbert, 50937 Köln (DE); Wirth, Andreas, 95466 Weidenberg (DE)
(74) Vertreter: Remus, Alvaro Johannes

(56) Entgegenhaltungen:
- EP-A- 1 577 378
- WO-A-03/057819
- WO-A-2005/044983
- WO-A-2007/056512
- WO-A-2007/094947
- WO-A1-03/049806
- US-A- 5 183 744
- US-A1- 2008 081 372
- US-B1- 6 878 538

## Beschreibung

Die Erfindung betrifft ein Verfahren zur Beaufschlagung von mindestens zwei mit Elektroden versehenen Behältnissen mit mindestens einem Spannungsimpuls, bei dem mindestens ein Behältnis mit mindestens einem Spannungsimpuls beaufschlagt wird, während mindestens ein anderes Behältnis einer Vor- oder Nachbereitung unterzogen wird. Die Erfindung betrifft ferner eine Vorrichtung zur elektrischen Kontaktierung mindestens eines mit Elektroden versehenen Behältnisses, mit mindestens einer Aufnahmevorrichtung zum Aufsetzen mindestens eines Behältnisses und mit mindestens einer Kontakteinrichtung zur Kontaktierung der Elektroden des Behältnisses.

Mit Elektroden versehene Behältnisse werden insbesondere bei Anwendungen eingesetzt, bei denen der Reaktionsansatz mit einem elektrischen Spannungsimpuls beaufschlagt werden muss, wie beispielsweise die Elektroporation, Elektrofusion und Elektrostimulation von lebenden Zellen. Solche Behältnisse können auch mehrere Reaktionsräume aufweisen, wobei jeder Reaktionsraum mit Elektroden versehen sein kann. Diese Behältnisse bezeichnet man in der Regel als Mehrlochplatten, Mikrotiterplatten oder Multiwells. Sie werden vor allem bei biochemischen und pharmazeutischen Anwendungen verwendet, bei denen eine Vielzahl von Reaktionsansätzen gleichzeitig getestet werden muss. Dabei ist das Bestreben eine möglichst große Anzahl von Reaktionsräumen, beispielsweise 384, zur Verfügung zu stellen insbesondere bei HT-Analysen (HT = high throughput) zu erkennen, da hier eine Vielzahl von Proben in möglichst kurzer Zeit getestet werden soll.

Die bekannten Behältnisse bestehen üblicherweise aus mehreren Reaktionsräumen, die jeweils zwei Elektroden aufweisen, welche mit dem Reaktionsansatz, beispielsweise einer Zellsuspension, im Reaktionsraum in Kontakt stehen. Die beiden Elektroden eines Reaktionsraums erzeugen bei Anlegen einer elektrischen Spannung im Inneren des Reaktionsraums ein elektrisches Feld, wobei sie beispielsweise bei Gleichstrom unterschiedliche Polaritäten und/oder Potentiale aufweisen. Die Elektroden gleicher Polarität, d. h. beispielsweise alle Kathoden und/oder alle Anoden, verschiedener Reaktionsräume sind dabei entweder einstückig ausgebildet oder elektrisch miteinander gekoppelt, so dass sie über einen gemeinsamen elektrischen Kontakt mit der Spannungsquelle verbunden werden können (siehe z.B. WO03/049806). Die Beaufschlagung der Reaktionsräume solcher Behältnisse mit Spannungsimpulsen erfolgt mittels besonderer Schaltungsanordnungen, die eine oder zwei Speichereinrichtung(en) zum Speichern elektrischer Ladungen umfassen. Bei den Speichereinrichtungen handelt es sich jeweils um Kondensatoren, die auf eine vorbestimmte elektrische Spannung aufgeladen werden und durch gezielte Entladung definierte Spannungsimpulse abgeben können. Die Speichereinrichtungen sind mit elektrischen Schaltern, verbunden, vorzugsweise Leistungshalbleitern, über welche die gezielte Entladung der Speichereinrichtungen geschaltet wird. Die Verwendung von zwei Speichereinrichtungen ermöglicht die Abgabe von zwei kurz aufeinander folgenden oder ineinander übergehenden Spannungsimpulsen, was bei der Elektroporation von bestimmten Zelltypen von Vorteil sein kann. Zur elektrischen Kontaktierung der Elektroden der Behältnisse dienen in der Regel Kontaktstifte, die auf Armen oder Platten angeordnet sind und manuell oder automatisch mit den Elektroden in Kontakt gebracht werden.

Aus der EP-A-1 577 378 sind ein Verfahren und eine Vorrichtung zur Kontaktierung eines aus mehreren Reaktionsräumen bestehenden Behältnisses bekannt, beispielsweise einer Mikrotiterplatte mit 96 Reaktionsräumen. Die Vorrichtung weist ein Gehäuse auf, in dem eine plattenförmige Kontakteinrichtung angeordnet ist, auf der stiftförmige Kontaktmittel befestigt sind. Die Kontakteinrichtung mit den Kontaktmitteln dient der elektrischen Kontaktierung der Elektroden des Behältnisses und ist vertikal bewegbar. Die Vorrichtung umfasst ferner eine tischförmige Aufnahmevorrichtung, auf die das Behältnis aufgesetzt werden kann und die horizontal bewegbar ist. Wird ein Behältnis auf die Aufnahmevorrichtung aufgesetzt, so kann die Aufnahmevorrichtung durch die Gehäuseöffnung in den Innenraum des Gehäuses gefahren werden. Daraufhin bewegt sich die Kontakteinrichtung von unten vertikal in Richtung des Behältnisses bis die Kontaktmittel durch Löcher in der Aufnahmevorrichtung hindurchragen und mit den Elektroden des Behältnisses in Kontakt treten.

Insbesondere bei Hochdurchsatz-Verfahren spielt die Gesamtzeit eines Behandlungszyklus eine entscheidende Rolle, da Zellen nur eine begrenzte Prozessdauer leben. Die Prozessdauer sollte daher möglichst gering ausfallen. In den bekannten Hochdurchsatz-Verfahren werden die Behältnisse mit den Reaktionsräumen von einem Roboter vorgefüllt geliefert und nach der elektrischen Behandlung von diesem Roboter weiter verarbeitet. Dabei muss der Roboter während der Behandlung eines Behältnisses warten, um das Behältnis dann gegen das nächste zu behandelnde Behältnis auszutauschen, wobei in dieser Zeit kein Behältnis der elektrischen Behandlung unterzogen werden kann.

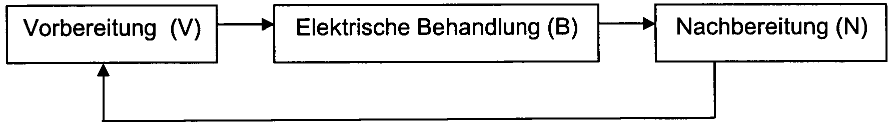

Die Prozessdauer pro Zyklus (T_{P} = V+B+N) ist also wesentlich von der Zeit (V+N) abhängig, die für das Austauschen der Behältnisse zwischen zwei Behandlungen (B) benötigt wird und nicht parallel zu den Behandlungen (B) durchgeführt werden kann.

Es ist Aufgabe der Erfindung, ein Verfahren und eine Vorrichtung zur elektrischen Kontaktierung von mindestens zwei Behältnissen mit verkürzter Prozessdauer zur Verfügung zu stellen.

Erfindungsgemäß wird die Aufgabe durch ein Verfahren der eingangs genannten Art gelöst, welches die folgenden Schritte umfasst:
a) Vorbereiten mindestens eines Behältnisses (4) außerhalb des Gehäuses (2) und Einbringen dieses Behältnisses (4) in das Gehäuse (2);
b) Beaufschlagen dieses Behältnisses (4) mit mindestens einem Spannungsimpuls innerhalb des Gehäuses (2) und gleichzeitig Vorbereiten mindestens eines weiteren Behältnisses (15) außerhalb des Gehäuses (2);
c) Gegenseitiges Tauschen der jeweiligen Positionen der Behältnisse (4, 15), wobei das bereits mit mindestens einem Spannungsimpuls beaufschlagte Behältnis (4) aus dem Gehäuse (2) heraus und das weitere Behältnis (15) in das Gehäuse hinein bewegt wird;
d) Beaufschlagen des weiteren Behältnisses (15) mit mindestens einem Spannungsimpuls innerhalb des Gehäuses (2) und gleichzeitig Nachbereiten des zuvor mit mindestens einem Spannungsimpuls beaufschlagten Behältnisses (4) sowie Vorbereiten mindestens eines nächsten Behältnisses außerhalb des Gehäuses (2).

Dadurch, dass immer mindestens ein Behältnis der elektrischen Behandlung unterzogen wird und gleichzeitig mindestens ein weiteres Behältnis vor- oder nachbereitet wird, kann die Prozessdauer insgesamt deutlich reduziert werden. D. h., während das Behältnis der elektrischen Behandlung unterzogen wird, kann ein Roboter das bereits behandelte Behältnis entfernen und das nächste zu behandelnde Behältnis vorbereiten. Die Prozessdauer pro Zyklus (T_{P}) wird durch das erfindungsgemäße Verfahren also um die Wartezeiten und die Bedienzeiten reduziert, so dass der Durchsatz insgesamt wesentlich erhöht werden kann.

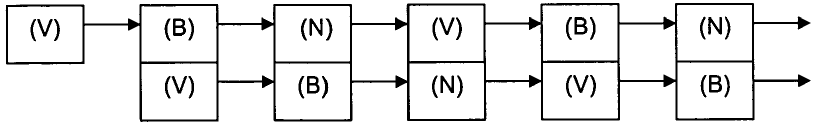

Erfindungsgemäß werden die Zyklen ineinander geschoben, so dass die Prozessdauer pro Zyklus (T_{P}) entweder B oder V+N ist, je nachdem welche Zeit länger ist. Das erfindungsgemäße Verfahren lässt sich mit mindestens zwei Behältnissen, insbesondere auch mehr als drei Behältnissen, durchführen. Im einfachsten Fall kann das erfindungsgemäße Verfahren beispielsweise durch einen Drehteller mit zwei Positionen umgesetzt werden.

Gegenseitiges Tauschen der jeweiligen Positionen der Behältnisse bedeutet im Sinne der Erfindung, dass die Positionen der Behältnisse relativ zueinander oder relativ zu anderen Bestandteilen einer Vorrichtung zur Durchführung des erfindungsgemäßen Verfahrens, beispielsweise eine den Spannungsimpuls abgebende Vorrichtung, geändert werden. D. h., dass entweder die Behältnisse relativ zueinander bewegt werden und/oder dass die anderen Bestandteile relativ zu den Behältnissen bewegt werden, wobei die Behältnisse nicht bewegt werden müssen.

Die Schritte b) bis d) werden vorzugsweise mehrfach wiederholt, wobei das Verfahren beendet wird, sobald alle zu behandelnden Behältnisse dem bzw. den Spannungsimpuls(en) beaufschlagt wurden.

In vorteilhafter Ausgestaltung des erfindungsgemäßen Verfahrens ist vorgesehen, dass die Behältnisse während der Schritte b) und d) jeweils mit mehreren Spannungsimpulsen beaufschlagt werden. Dies ist insbesondere erforderlich, wenn Behältnisse mit mehreren Reaktionsräumen, insbesondere 384 Reaktionsräumen, behandelt werden müssen. Da in diesem Fall die Behandlung des Behältnisses eine längere Zeit in Anspruch nimmt, ist es besonders von Vorteil, dass das Vorbereiten des nächsten Behältnisses zeitgleich stattfindet, so dass auch bei Anwendungen, bei denen sehr viele Proben behandelt werden müssen, die Prozesszeit relativ kurz gehalten werden kann.

Vorzugsweise umfasst das Vorbereiten der Behältnisse während der Schritte a), b) und d) das Zuführen der Behältnisse und/oder das Nachbereiten des Behältnisses während Schritt d) das Entfernen des Behältnisses.

Das Tauschen der Positionen der Behältnisse während Schritt c) kann beispielsweise derart erfolgen, dass die Behältnisse gegeneinander ausgetauscht werden. Gleichzeitig oder alternativ kann das Tauschen der Positionen der Behältnisse während Schritt c) auch derart erfolgen, dass eine den Spannungsimpuls abgebende Vorrichtung in die jeweils andere Position bewegt wird. Dabei kann das Austauschen der Positionen beispielsweise mittels einer Drehbewegung erfolgen. Insbesondere können die Bewegungen der Behältnisse dabei gekoppelt sein, d. h. gleichzeitig erfolgen. Alternativ kann das Austauschen der Positionen mittels einer linearen Bewegung erfolgen. Insbesondere können die Bewegungen der Behältnisse dabei entkoppelt sein, d. h. zumindest teilweise nacheinander erfolgen.

In besonders vorteilhafter Ausgestaltung des erfindungsgemäßen Verfahrens ist vorgesehen, dass die Elektroden der Behältnisse während der Schritte b) und d) mittels mindestens einer Kontakteinrichtung kontaktiert werden, wobei die Kontakteinrichtung im Wesentlichen annähernd parallel zur Ebene der Kontaktierung, vorzugsweise horizontal, über dem jeweiligen Behältnis bewegt wird. Die Elektroden eines Behältnisses können somit von einer Kontakteinrichtung nacheinander abgefahren werden, wobei die Kontakteinrichtung vorzugsweise schrittweise über das Behältnis fährt. In weiterer vorteilhafter Ausgestaltung des erfindungsgemäßen Verfahrens ist vorgesehen, dass die Elektroden der Behältnisse während der Schritte b) und d) mittels mindestens einer mit mindestens einem Kontaktelement versehenen Kontakteinrichtung kontaktiert werden, wobei das Kontaktelement zum Herstellen des Kontakts im Wesentlichen annähernd senkrecht zur Ebene der Kontaktierung, vorzugsweise vertikal, bewegt wird. Die Kontakteinrichtung kann also beispielsweise horizontal über das Behältnis fahren, wobei der elektrische Kontakt dann bei Stillstand der Kontakteinrichtung durch eine vertikale Bewegung der Kontaktmittel in Richtung der Elektroden hergestellt wird.

Die Aufgabe wird ferner durch eine Vorrichtung wie beansprucht gelöst, bei der mindestens zwei Aufnahmevorrichtungen vorgesehen sind, wobei sich eine Aufnahmevorrichtung an oder innerhalb der Kontakteinrichtung befindet und wobei beide Aufnahmevorrichtungen und/oder die Kontakteinrichtung derart bewegbar sind, dass sich die andere Aufnahmevorrichtung nach Abschluss der Bewegung an oder innerhalb der Kontakteinrichtung befindet. Die Aufnahmevorrichtungen mit den Behältnissen sind erfindungsgemäß relativ zur Kontakteinrichtung bewegbar, wobei Sie ihre Positionen tauschen können. Hierdurch wird beispielsweise ermöglicht, eine Aufnahmevorrichtung, die ein bereits elektrisch kontaktiertes bzw. behandeltes Behältnis trägt, von der Kontakteinrichtung wegzubewegen und gleichzeitig eine Aufnahmevorrichtung, die ein noch elektrisch zu kontaktierendes bzw. zu behandelndes Behältnis trägt, an oder in die Kontakteinrichtung zu bewegen. Das Tauschen der Positionen der Aufnahmevorrichtungen stellt eine sehr schnelle Möglichkeit dar, für den überwiegenden Teil der Prozesszeit ein Behältnis elektrisch zu kontaktieren bzw. zu behandeln und gleichzeitig mindestens ein anderes Behältnis vor- bzw. nachzubereiten. Während also die Elektroden mindestens eines Behältnisses durch die Kontakteinrichtung elektrisch kontaktiert werden, ist mindestens eine andere Aufnahmevorrichtung frei zugänglich, so dass hier ein Behältnis abgehoben und das nächste zur Behandlung anstehende Behältnis auf die Aufnahmevorrichtung aufgesetzt werden kann. Die erfindungsgemäße Vorrichtung ist also sehr schnell und kann viele Behältnisse mit hohen Durchsatzraten prozessieren.

Die Aufnahmevorrichtungen können beispielsweise auf einer beweglichen Platte angeordnet sein. Die bewegliche Platte ist vorzugsweise radial bewegbar und kann beispielsweise ein Drehteller sein. Alternativ kann die bewegliche Platte aber auch linear bewegbar sein.

In besonders vorteilhafter Ausgestaltung der erfindungsgemäßen Vorrichtung ist vorgesehen, dass mindestens eine Aufnahmevorrichtung unterhalb der Kontakteinrichtung angeordnet ist, so dass die Elektroden eines auf der Aufnahmevorrichtung angeordneten Behältnisses von oben kontaktiert werden können. Alternativ kann die Aufnahmevorrichtung auch oberhalb der Kontakteinrichtung angeordnet sein, insbesondere dann, wenn die Behältnisse nur von unten kontaktierbar sind.

Vorzugsweise ist die Kontakteinrichtung innerhalb eines Gehäuses angeordnet, so dass keine Verletzungsgefahr für die Bedienperson(en) durch die elektrischen Spannungsimpulse besteht. Vorzugsweise sind dabei mindestens eine Aufnahmevorrichtung innerhalb des Gehäuses und mindestens eine andere Aufnahmevorrichtung außerhalb des Gehäuses angeordnet, so dass die andere Aufnahmevorrichtung frei zugänglich bleibt. Wenn die Aufnahmevorrichtungen auf einer beweglichen Platte angeordnet sind, sollten dabei ein Teil der beweglichen Platte innerhalb des Gehäuses und ein anderer Teil der beweglichen Platte außerhalb des Gehäuses angeordnet sein.

Die Kontakteinrichtung kann bei einer alternativen Ausführungsform der erfindungsgemäßen Vorrichtung zwischen den Aufnahmevorrichtungen hin und her bewegbar sein, so dass die Positionen der Aufnahmevorrichtungen relativ zur Kontakteinrichtung im Sinne der Erfindung schnell gegeneinander ausgetauscht werden können.

Die Erfindung betrifft auch ein Verfahren zur elektrischen Kontaktierung mindestens eines Behältnisses, das mindestens zwei mit jeweils mindestens einer Elektrode versehene Reaktionsräume umfasst, wobei die Elektroden verschiedener Reaktionsräume sequenziell elektrisch kontaktiert werden. Der elektrische Kontakt zu den Elektroden der zu behandelnden Reaktionsräume wird erfindungsgemäß also nacheinander und nicht gleichzeitig hergestellt. Dieses vorteilhafte Verfahren ermöglicht in vorteilhafter Weise, dass die mechanische Belastung durch die Kontaktierung für das Behältnis sowie die Aufnahmevorrichtung und die Kontakteinrichtung deutlich verringert wird, da immer nur ein Teil der Elektroden eines Behältnisses gleichzeitig kontaktiert wird. Die Summe aller auf das Behältnis wirkenden Kräfte würde nämlich bei gleichzeitiger Kontaktierung aller Elektroden des zu kontaktierenden Behältnisses zu einer hohen mechanischen Belastung führen. Die geringe Anzahl an erforderlichen Kontaktmitteln zur Herstellung des elektrischen Kontakts hat auch den Vorteil, dass das Ausfallrisiko sowie die Kosten für die Kontakteinrichtung erheblich reduziert werden.

In vorteilhafter Ausgestaltung des erfindungsgemäßen Verfahrens ist vorgesehen, dass eine Kontakteinrichtung zur elektrischen Kontaktierung der Elektroden annähernd parallel zu mindestens einer Ebene der Kontaktierung bewegt und dann der elektrische Kontakt zu den Elektroden hergestellt wird. Dieses vorteilhafte Verfahren ermöglicht das schnelle Abfahren der Oberfläche bzw. der Elektroden eines Behältnisses, wobei der elektrische Kontakt beispielsweise durch eine möglichst schnelle und kurze Bewegung der Kontakteinrichtung oder eines Teils der Kontakteinrichtung in Richtung des Behältnisses hergestellt werden kann.

In weiterer vorteilhafter Ausgestaltung des erfindungsgemäßen Verfahrens ist vorgesehen, dass die Kontakteinrichtung im Wesentlichen annähernd parallel zur Ebene der Kontaktierung, vorzugsweise horizontal, über dem Behältnis bewegt wird. Der elektrische Kontakt wird dann vorzugsweise durch eine im Wesentlichen annähernd senkrecht zur Ebene der Kontaktierung, vorzugsweise vertikal, verlaufende Bewegung zumindest eines Teils der Kontakteinrichtung hergestellt.

In besonders vorteilhafter Ausgestaltung des erfindungsgemäßen Verfahrens ist vorgesehen, dass der Anpressdruck, mit dem die Elektroden kontaktiert werden, begrenzt wird. Dies kann elektronisch und/oder durch eine Reduzierung der Kontaktpunkte erfolgen. Darüber hinaus können gefederte Kontaktmittel verwendet werden.

Für den Ausgleich von Toleranzen der Kontaktmittel kann es erforderlich sein, gefederte Kontaktmittel, beispielsweise Kontaktstifte mit gefedertem Kopf, zu verwenden. Die Summe aller erforderlichen Kontaktmittel mit jeweiliger Federkraft führen aber zu hoher Anpresskraft bei Kontaktierung aller Elektroden eines zu kontaktierenden Behältnisses, unabhängig von der Kontaktierrichtung.

Um die Prozessdauer zu minimieren, werden vorzugsweise mehrere Elektroden, besonders bevorzugt die Elektroden von mindestens zwei Reaktionsräumen, gleichzeitig kontaktiert. In diesem Fall sollte die mechanische Belastung für das aufnehmende Gerät und das Behältnis verringert werden, insbesondere wenn sehr viele Elektroden gleichzeitig kontaktiert werden müssen, beispielsweise bei der Verwendung von 96- oder 384-well-Platten in Hochdurchsatzverfahren. Außerdem bedeutet die hohe Anzahl an erforderlichen Kontaktiernadeln auch ein erhöhtes Ausfallrisiko sowie nicht unerhebliche Kosten der Kontakteinrichtung. Diese Probleme können durch eine sequentielle Kontaktierung einzelner Gruppen von Reaktionsräumen bzw. verschiedener Elektroden eines Behältnisses mittels einer verfahrbaren Kontakteinrichtung gelöst werden. Insbesondere können auch die Elektroden einer oder mehrerer Reihen von Reaktionsräumen, vorzugsweise zwei Reihen, gleichzeitig kontaktiert und unterschiedliche Reihen oder Reihengruppen von Reaktionsräumen sequenziell kontaktiert werden. Wenn also das Behältnis mehrere Reaktionsräume mit jeweils mindestens einer Elektrode aufweist, wobei einzelne Gruppen von Reaktionsräumen festgelegt werden, und die Elektroden der Reaktionsräume einer Gruppe gleichzeitig und die Elektroden der Reaktionsräume verschiedener Gruppen nacheinander kontaktiert werden, dann kann der Anpressdruck und somit die mechanische Belastung für die Vorrichtung effektiv reduziert werden.

Die Erfindung betrifft ferner eine Vorrichtung zur elektrischen Kontaktierung mindestens eines Behältnisses, das mindestens zwei mit jeweils mindestens einer Elektrode versehene Reaktionsräume umfasst, wobei mindestens eine Kontakteinrichtung vorgesehen ist, welche mindestens eine Kontakteinheit umfasst, die Kontaktmittel zur Kontaktierung der Elektroden aufweist, und wobei die Anzahl der Kontakteinheiten geringer als die Anzahl der Reaktionsräume ist, wobei eine Kontakteinheit Kontaktmittel zur Kontaktierung der Elektroden mindestens zweier Reaktionsräume aufweist. Die Summe der Anpresskräfte aller zur Herstellung des elektrischen Kontakts erforderlichen Kontaktmittel würde bei einer Kontaktierung aller Elektroden des zu kontaktierenden Behältnisses zu einem insgesamt sehr hohen Anpressdruck führen. Die mechanische Belastung für die Aufnahmevorrichtung, die Kontakteinrichtung und das Behältnis wird daher erfindungsgemäß dadurch verringert, dass die Anzahl der Kontakteinheiten und damit auch der Kontaktmittel geringer als die Anzahl der Reaktionsräume ist. Die geringe Anzahl an Kontaktmitteln hat auch den Vorteil, dass das Ausfallrisiko sowie die Kosten für die Kontakteinrichtung erheblich reduziert werden.

Vorzugsweise ist die Kontakteinrichtung trägerartig, insbesondere brücken-und/oder schlittenartig, ausgebildet und im Wesentlichen annähernd parallel zur Ebene der Kontaktierung, vorzugsweise horizontal, bewegbar.

In vorteilhafter Ausgestaltung der Erfindung ist vorgesehen, dass zumindest ein Teil der Kontakteinrichtung der erfindungsgemäßen Vorrichtung im Wesentlichen annähernd senkrecht zur Ebene der Kontaktierung, vorzugsweise vertikal, bewegbar ist. Dieser Teil der Kontakteinrichtung umfasst vorzugsweise die Kontakteinheit und/oder die Kontaktmittel, mittels derer die Elektroden der Behältnisse kontaktiert werden.

Vorzugsweise umfassen mindestens zwei Kontakteinheiten mindestens ein gemeinsames Kontaktelement, so dass in vorteilhafter Weise Behältnisse kontaktiert werden können, die Reaktionsräume umfassen, welche zumindest teilweise gemeinsame und/oder elektrisch gekoppelte Elektroden aufweisen.

Die Kontakteinrichtung kann also Kontaktmittel umfassen, die vorzugsweise Kontaktstifte oder -nadeln sind. Die Kontaktmittel können gefedert gelagert sein.

In vorteilhafter Ausgestaltung der Erfindung ist ferner vorgesehen, dass mehrere Kontakteinheiten in mindestens einer Reihe auf der Kontakteinrichtung angeordnet sind. Dabei sind mindestens drei Reihen von Kontaktmitteln vorgesehen, so dass die Kontakteinheiten derart auf der Kontakteinrichtung angeordnet sein müssen, dass mindestens drei Reihen von Kontaktmitteln auf der Kontakteinrichtung angeordnet sein können. Dabei können die Kontaktmittel auch zickzackförmig angeordnet sein.

In weiterer vorteilhafter Ausgestaltung der Erfindung ist vorgesehen, dass die Kontakteinrichtung innerhalb einer rahmenartigen Befestigungsvorrichtung befestigt ist, welche vorzugsweise im Wesentlichen annähernd senkrecht zur Ebene der Kontaktierung, insbesondere vertikal, bewegbar ist.

Die Kontakteinrichtung kann auf Führungselementen im Wesentlichen annähernd parallel zur Ebene der Kontaktierung, vorzugsweise horizontal, bewegbar gelagert sein.

Die Erfindung betrifft auch ein Verfahren zur elektrischen Kontaktierung mindestens eines mit Elektroden versehenen Behältnisses, bei dem das Behältnis durch eine Kühleinrichtung gekühlt wird, wobei das Behältnis annähernd gleichmäßig auf die Kühleinrichtung aufgepresst wird. Durch das gleichmäßige Aufpressen wird ein gleichmäßiger, effektiver und schneller Wärmeübergang von den Elektroden bzw. der Oberfläche des Behältnisses auf die Kühleinrichtung gewährleistet.

Vorzugsweise wird der Druck, mit dem das Behältnis auf die Kühleinrichtung aufgepresst wird, zumindest teilweise auf die Ränder des Behältnisses ausgeübt. Zusätzlich oder alternativ kann der Druck, mit dem das Behältnis auf die Kühleinrichtung aufgepresst wird, auch zumindest teilweise auf die Elektroden des Behältnisses ausgeübt werden, beispielsweise durch die Kontaktmittel. Dabei sollte das Behältnis derart auf die Kühleinrichtung aufgepresst werden, dass zwischen dem Behältnis und der Kühleinrichtung befindliche Luft weitgehend verdrängt wird.

Das erfindungsgemäße Verfahren eignet sich insbesondere für den Einsatz bei der elektrischen Behandlung von Behältnissen mit mehreren Reaktionsräumen, beispielsweise 384 Reaktionsräumen.

Die Erfindung betrifft ferner eine Vorrichtung zur elektrischen Kontaktierung mindestens eines mit Elektroden versehenen Behältnisses, mit mindestens einer Aufnahmevorrichtung zum Aufsetzen mindestens eines Behältnisses, wobei die Aufnahmevorrichtung mit einer Kühleinrichtung versehen ist.

Die Kühleinrichtung umfasst dabei vorzugsweise mindestens ein Peltier-Element. Alternativ oder zusätzlich kann die Kühleinrichtung aber auch mindestens einen Kühlkörper und/oder Kältespeicher und/oder mindestens einen Ventilator umfassen.

Die erfindungsgemäße Vorrichtung kann in vorteilhafter Ausgestaltung der Erfindung mindestens eine Anpresseinrichtung umfassen, welche das Behältnis auf die Aufnahmevorrichtung und/oder die Kühleinrichtung presst. Die Anpresseinrichtung kann rahmenförmig ausgebildet und zumindest auf einen Teil des Randes des Behältnisses aufsetzbar sein.

In besonders vorteilhafter Ausgestaltung der Erfindung ist vorgesehen, dass die Anpresseinrichtung eine elektrische Kontakteinrichtung ist.

Das Behältnis kann mehrere Reaktionsräume, vorzugsweise 96 oder 384 Reaktionsräume, umfassen.

Die Erfindung wird im Weiteren anhand der Figuren beispielhaft näher erläutert.
Figur 1 zeigt eine perspektivische Darstellung einer Ausführungsform einer erfindungsgemäßen Vorrichtung mit Gehäuse und Behältnis.
Figur 2 zeigt eine perspektivische Darstellung der erfindungsgemäßen Vorrichtung gemäß Figur 1 ohne Gehäuse und Behältnis.
Figur 3 zeigt eine Draufsicht auf die erfindungsgemäße Vorrichtung gemäß Figur 2.
Figur 4 zeigt eine Seitenansicht der erfindungsgemäßen Vorrichtung gemäß Figur 2.
Figur 5 zeigt eine Seitenansicht einer Ausführungsform einer erfindungsgemäßen Vorrichtung mit einem Behältnis und einer Kontakteinrichtung.
Figur 6 zeigt eine schematische Seitenansicht einer Ausführungsform einer erfindungsgemäßen Vorrichtung mit einem Behältnis und einer Kühleinrichtung.
Figur 7 zeigt eine schematische Seitenansicht einer weiteren Ausführungsform einer erfindungsgemäßen Vorrichtung mit linearer Bewegung zweier Aufnahmevorrichtungen.

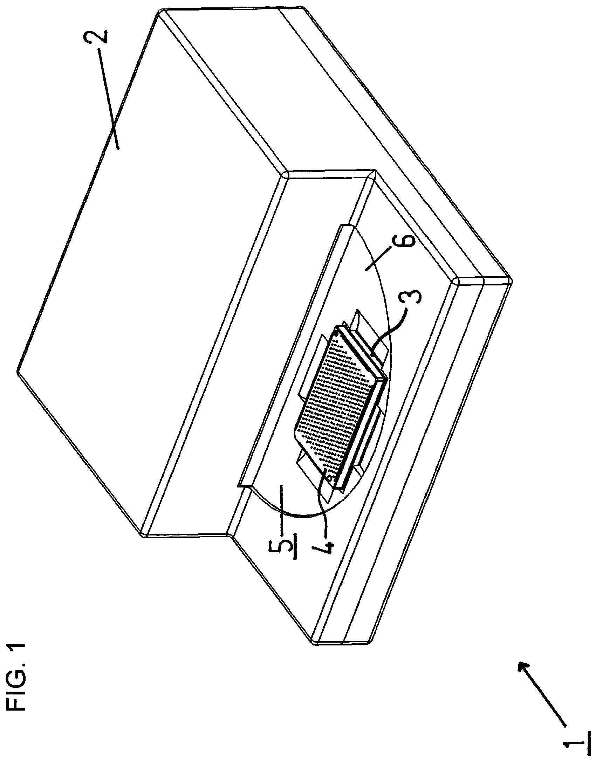

Figur 1 zeigt eine perspektivische Darstellung einer besonders vorteilhaften Ausführungsform einer erfindungsgemäßen Vorrichtung. Die Vorrichtung 1 weist ein Gehäuse 2 auf, in dem eine hier nicht sichtbare Aufnahmevorrichtung unterhalb einer ebenfalls nicht sichtbaren Kontakteinrichtung angeordnet ist. Die Vorrichtung 1 umfasst ferner eine weitere Aufnahmevorrichtung 3, auf die ein Behältnis 4 aufgesetzt ist. Bei dem Behältnis 4 handelt es sich in diesem Ausführungsbeispiel um eine Mikrotiterplatte mit 384 Reaktionsräumen, d. h. eine sogenannte 384-well-Platte. Es könnte sich alternativ aber auch um eine Mikrotiterplatte mit weniger oder mehr Reaktionsräumen oder ein anderes Behältnis mit mindestens einem Reaktionsraum handeln. Die Aufnahmevorrichtung 3 ist auf einer beweglichen Platte 5 angeordnet, welche bei dieser Ausführungsform in Form eines Drehtellers 6 ausgebildet ist. Da auch die nicht sichtbare Aufnahmevorrichtung auf dem Drehteller 6 angeordnet ist, können die Positionen der beiden Aufnahmevorrichtungen der erfindungsgemäßen Vorrichtung 1 durch einfache Drehung des Drehtellers 6 gegeneinander ausgetauscht werden. Wird also das Behältnis auf der nicht sichtbaren Aufnahmevorrichtung innerhalb des Gehäuses 2 einer elektrischen Behandlung durch das Beaufschlagen eines oder mehrerer Reaktionsräume mit mindestens einem Spannungsimpuls unterzogen, so kann gleichzeitig ein bereits behandeltes Behältnis von der außerhalb des Gehäuses liegenden Aufnahmevorrichtung 3 genommen und dann ein weiteres, noch zu behandelndes Behältnis, hier das Behältnis 4, auf die Aufnahmevorrichtung 3 aufgesetzt werden. Durch die radiale Bewegung des Drehtellers 6 kann dann das zuvor elektrisch behandelte Behältnis gegen das noch zu behandelnde Behältnis ausgetauscht werden.

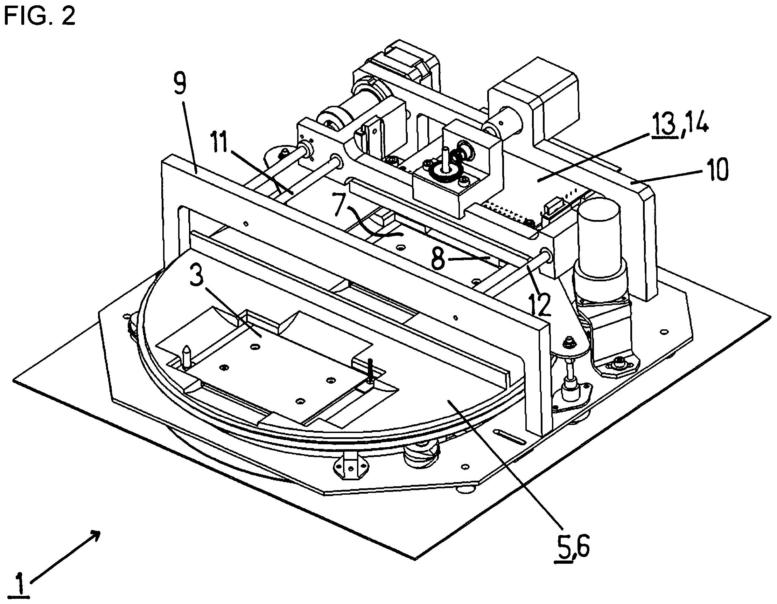

Figur 2 zeigt eine perspektivische Darstellung der Ausführungsform gemäß Figur 1 ohne Gehäuse und Behältnis. Die Vorrichtung 1 umfasst die bewegliche Platte 5, die in Form des Drehtellers 6 ausgebildet ist. Auf dem Drehteller 6 sind zwei Aufnahmevorrichtungen 3, 7 angeordnet. Die Aufnahmevorrichtung 7 ist unterhalb einer Kontakteinrichtung 8 angeordnet. Die Kontakteinrichtung 8 weist hier nicht sichtbare Kontaktmittel auf, welche die Elektroden der Behältnisse, die auf die Aufnahmevorrichtungen 3, 7 aufgesetzt sein können, kontaktieren können. Die Kontakteinrichtung 8 ist unterhalb einer Verteileinrichtung 13 angeordnet, die in diesem Ausführungsbeispiel eine Abdeckplatte 14 und darüber angeordnete Schaltelemente, vorzugsweise Relais (nicht dargestellt), umfasst. Mit Hilfe der Schaltelemente bzw. Relais werden die Spannungsimpulse auf die jeweiligen Kontaktmittel verteilt. Da beide Aufnahmevorrichtungen 3, 7 auf dem Drehteller 6 angeordnet sind, können die Positionen der beiden Aufnahmevorrichtungen 3, 7 durch eine einfache radiale Bewegung, d. h. Drehung, des Drehtellers 6 gegeneinander ausgetauscht werden. Wird also ein Behältnis auf der Aufnahmevorrichtung 7 einer elektrischen Behandlung durch das Beaufschlagen der Reaktionsräume dieses Behältnisses mit mindestens einem Spannungsimpuls unterzogen, so kann gleichzeitig ein bereits behandeltes Behältnis von der außerhalb des Gehäuses liegenden Aufnahmevorrichtung 3 genommen und dann ein weiteres, noch zu behandelndes Behältnis auf die Aufnahmevorrichtung 3 aufgesetzt werden. Durch Drehen des Drehtellers 6 kann dann das zuvor elektrisch behandelte Behältnis gegen das noch zu behandelnde Behältnis ausgetauscht werden. Die Kontakteinrichtung 8 ist in diesem Ausführungsbeispiel schlitten- bzw. brückenartig ausgebildet und an feststehenden Bügeln 9, 10 auf Schienen 11, 12 horizontal beweglich gelagert. Die Kontakteinrichtung 8 kann parallel zur Oberfläche des Behältnisses bzw. der Kontaktierebene bewegt werden. Die Kontakteinrichtung 8 kann also beispielsweise horizontal, vorzugsweise schrittweise, über die Aufnahmevorrichtung 7 bzw. das darauf befindliche Behältnis fahren, wobei der elektrische Kontakt dann bei Stillstand der Kontakteinrichtung 8 durch eine vertikale Bewegung mindestens einer Kontakteinheit bzw. der Kontaktmittel und/oder der gesamten Kontakteinrichtung 8 in Richtung der Elektroden des Behältnisses hergestellt werden kann.

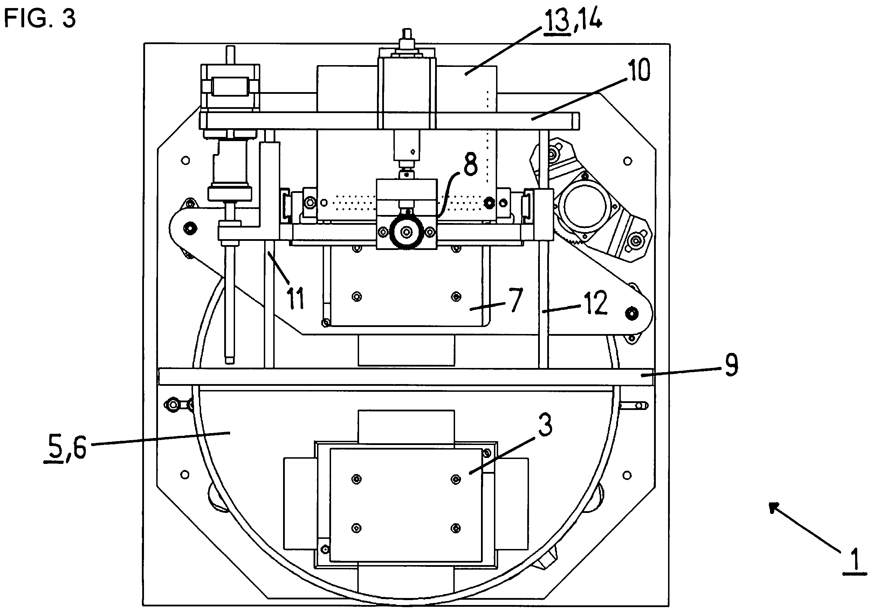

Figur 3 zeigt eine Draufsicht auf die erfindungsgemäße Vorrichtung gemäß Figur 2. Bei dieser Darstellung wird deutlich, dass die Positionen der Aufnahmevorrichtungen 3, 7 durch eine einfache radiale Bewegung der beweglichen Platte 5, d. h. in diesem Ausführungsbeispiel eine Drehung des Drehtellers 6, gegeneinander ausgetauscht werden können. Durch die radiale Bewegung der beweglichen Platte 5 kann die an bzw. teilweise unter der Kontakteinrichtung 8 angeordnete Aufnahmevorrichtung 7 auf einfache Weise sehr schnell von der Kontakteinrichtung 8 weg, in Richtung der gegenwärtigen Position der Aufnahmevorrichtung 3 außerhalb des hier nicht dargestellten Gehäuses bewegt werden. Da die Bewegungen der beiden Aufnahmevorrichtungen 3, 7 gekoppelt sind, würde in diesem Fall die Aufnahmevorrichtung 3 in das Gehäuse in Richtung der Kontakteinrichtung 8 transportiert werden. Nach Abschluss der radialen Bewegung, d. h. vorzugsweise nach einer Drehung um 180°, wäre dann die Aufnahmevorrichtung 3 an bzw. teilweise unter der Kontakteinrichtung 8 angeordnete, während sich die Aufnahmevorrichtung 7 außerhalb des Gehäuses befinden würde. Da beispielsweise bei Elektrotransfektionen im Hochdurchsatzverfahren (high throughput screening) die Gesamtzeit eines Transfektionszyklus eine entscheidende Rolle spielt, weil die Zellen nur eine begrenzte Prozessdauer leben, ist das erfindungsgemäße Verfahren bzw. die erfindungsgemäße Vorrichtung besonders vorteilhaft. Die Prozessdauer wird nämlich durch das erfindungsgemäße Verfahren mit Hilfe der erfindungsgemäßen Vorrichtung um die Wartezeiten und die Bedienzeiten reduziert und somit der Durchsatz wesentlich erhöht. In Hochdurchsatz-Transfektionen werden die Mikrotiterplatten mit den Reaktionsräumen von einem Roboter vorgefüllt geliefert und nach der Transfektion von diesem Roboter weiter verarbeitet. D. h., während innerhalb des Gehäuses die elektrische Behandlung durchgeführt wird, kann erfindungsgemäß außen der Roboter das letzte, bereits behandelte Behältnis entfernen und das neue Behältnis vorbereiten. Es geht also keine Zeit für die Vor- und Nachbereitung der Behältnisse zwischen den elektrischen Behandlungen verloren, so dass die Prozessdauer insgesamt erheblich reduziert werden kann. Während der elektrischen Behandlung wird dabei die unterhalb der Verteileinrichtung 13 angeordnete Kontakteinrichtung 8 horizontal, d. h. parallel zur Kontaktierebene, über dem Behältnis, das sich auf der Aufnahmevorrichtung 7 befindet, bewegt. Vorzugsweise wird dabei das Behältnis schrittweise abgefahren, wobei die brückenartige Kontakteinrichtung 8 entlang der Schienen 11, 12 geführt wird. Wenn die Kontakteinrichtung 8 die gewünschte Position über dem Behältnis bzw. den zu behandelnden Reaktionsräumen erreicht hat, kann der elektrische Kontakt zwischen den Kontaktmitteln und den Elektroden hergestellt werden. Nachdem die entsprechenden Reaktionsräume mit dem oder den Spannungsimpuls(en) beaufschlagt wurden, wird der Kontakt zwischen den Kontaktmitteln und den Elektroden wieder getrennt und dann die Kontakteinrichtung 8 in die nächste Position bewegt, wo dann die nächsten Reaktionsräume behandelt werden. Diese Vorgänge können so lange wiederholt werden, bis alle gewünschten Reaktionsräume des Behältnisses behandelt wurden. Die Behältnisse werden dann wie oben beschrieben gegeneinander ausgetauscht.

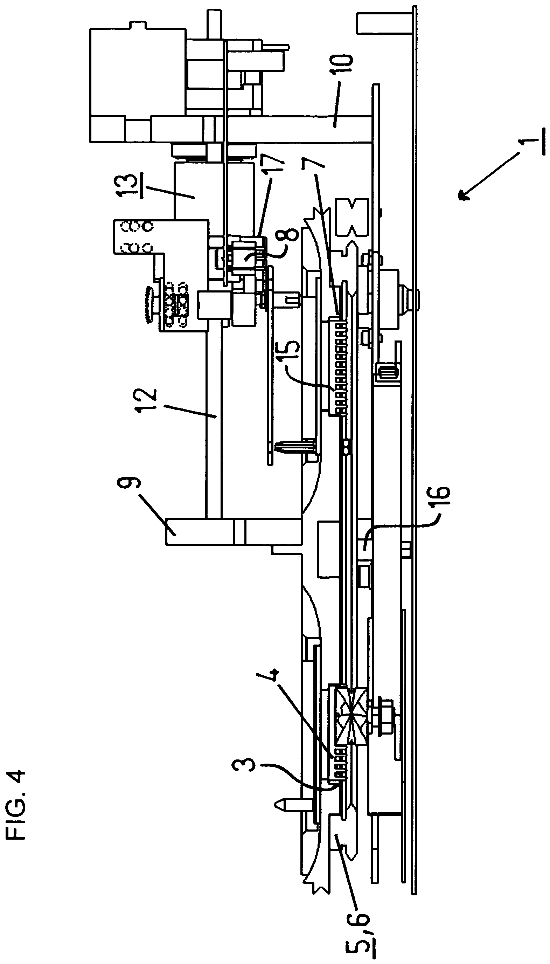

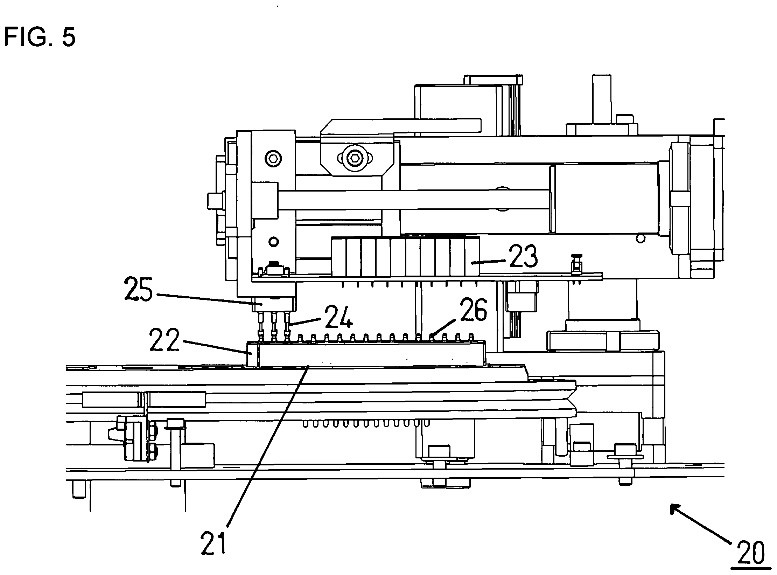

Figur 4 zeigt eine Seitenansicht der Ausführungsform gemäß Figur 2. Es wird hier deutlich, dass die bewegliche Platte 5 eine radiale bzw. kreisförmige Bewegung um die Achse 16 vollführen kann, so dass die Positionen der Aufnahmevorrichtungen 3, 7 gegeneinander ausgetauscht werden können. Es wird hier ferner deutlich, dass zwei Behältnisse 4, 15 auf der beweglichen Platte 5 angeordnet sind. Das Behältnis 4 ist auf die Aufnahmevorrichtung 3 außerhalb des hier nicht dargestellten Gehäuses aufgesetzt, während das Behältnis 15 auf die Aufnahmevorrichtung 7 innerhalb des Gehäuses aufgesetzt ist. Das Behältnis 15 ist also an der Kontakteinrichtung 8 angeordnet, die entlang der Schiene 12 über dem Behältnis 15 horizontal bewegt werden kann. Diese Bewegung und die Herstellung des elektrischen Kontakts zwischen den Kontaktmitteln 17 der Kontakteinrichtung 8 und den Elektroden des Behältnisses 15 werden anhand der Figur 5 näher beschrieben.

Figur 5 zeigt eine Seitenansicht einer Ausführungsform einer erfindungsgemäßen Vorrichtung mit einem Behältnis und einer Kontakteinrichtung. Die Vorrichtung 20 umfasst eine Aufnahmevorrichtung 21, auf der ein Behältnis 22 angeordnet ist. Das Behältnis 22 umfasst mehrere Reaktionsräume, die jeweils mit zwei Elektroden versehen sind, die über aus den Elektroden nach oben herausragende Kontaktelemente 26 von oben elektrisch kontaktiert werden können. Die Kontaktierung erfolgt mittels der Kontaktmittel 24, die an der Unterseite einer Kontakteinrichtung 25 befestigt und in diesem Beispiel stiftförmig ausgebildet sind. Die Kontakteinrichtung 25 wird horizontal, d. h. parallel zur Kontaktierebene, über dem Behältnis 22 bewegt. Vorzugsweise wird dabei das Behältnis schrittweise abgefahren. Wenn die Kontakteinrichtung 25 die gewünschte Position über dem Behältnis 22 bzw. den zu behandelnden Reaktionsräumen erreicht hat, kann der elektrische Kontakt zwischen den Kontaktmitteln 24 und den Kontaktelementen 26 der Elektroden der jeweiligen Reaktionsräume hergestellt werden. Der Kontakt wird dabei durch eine vertikale Bewegung, d. h. ein Absenken, der Kontakteinrichtung 25 in Richtung des Behältnisses 22 hergestellt. Zum Ausgleich von Toleranzen der Kontaktmittel ist es vorteilhaft, Kontaktmittel mit gefedertem Kopf zu verwenden. Die Summe aller erforderlichen Kontaktmittel mit jeweiliger Federkraft würde aber bei Kontaktierung aller Elektroden des zu kontaktierenden Behältnisses zu einer hohen Anpresskraft führen, unabhängig von der Kontaktierrichtung. Die mechanische Belastung für die Aufnahmevorrichtung und das Behältnis wird erfindungsgemäß dadurch verringert, dass immer nur ein Teil der Elektroden eines Behältnisses gleichzeitig kontaktiert wird. Die Kontakteinrichtung 25 ist daher brückenförmig ausgebildet und weist an seiner Unterseite drei Reihen von Kontaktmitteln auf (von denen hier nur die vordersten Kontaktmittel 24 sichtbar sind), deren Gesamtzahl geringer als die Anzahl der Reaktionsräume ist. Die geringe Anzahl an erforderlichen Kontaktmitteln hat auch den Vorteil, dass das Ausfallrisiko sowie die Kosten für die Kontakteinrichtung erheblich reduziert werden. Erfindungsgemäß erfolgt also in vorteilhafter Weise eine sequentielle Kontaktierung jeweils eines Teils des Behältnisses 22, vorzugsweise von zwei Reihen von Reaktionsräumen, mittels der verfahrbaren Kontakteinrichtung 25. Aufgrund der Form des Behältnisses 22, das in diesem Ausführungsbeispiel eine Mikrotiterplatte ist, ist dabei eine Anordnung der Kontaktmittel 24 in Reihen vorteilhaft. Nachdem die entsprechenden Reaktionsräume mit dem oder den Spannungsimpuls(en) beaufschlagt wurden, wird der Kontakt zwischen den Kontaktmitteln 24 und den Kontaktelementen 26 der Elektroden wieder getrennt und dann die Kontakteinrichtung 25 in die nächste Position bewegt, wo dann die nächsten Reaktionsräume behandelt werden. Diese Vorgänge können so lange wiederholt werden, bis alle gewünschten Reaktionsräume des Behältnisses 22 behandelt wurden. Die Spannungsimpulse werden dabei mittels einer Verteileinrichtung 23, die beispielsweise einzelne Relais umfassen kann, auf die einzelnen Kontaktmittel 24 verteilt.

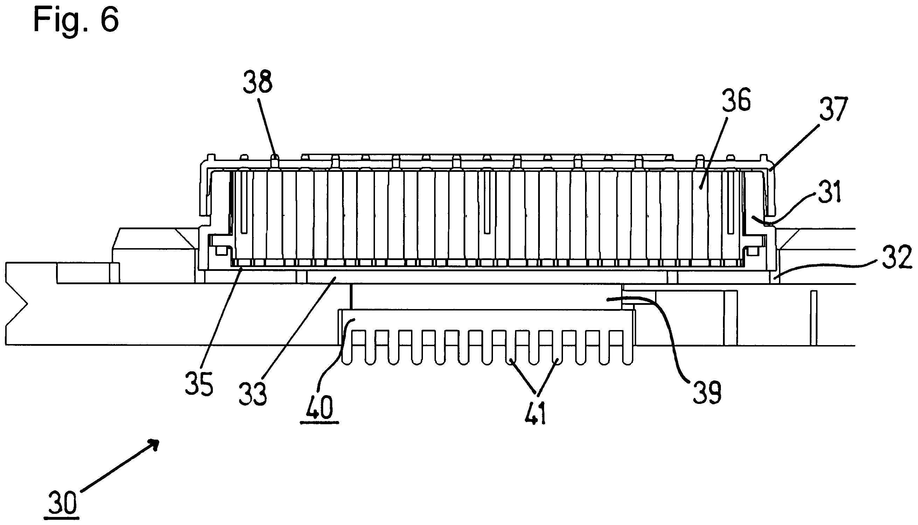

Figur 6 zeigt eine schematische Seitenansicht einer Ausführungsform einer erfindungsgemäßen Vorrichtung 30 zur elektrischen Kontaktierung mindestens eines mit Elektroden 36 versehenen Behältnisses 31, das mit einem Deckel 37 versehen ist, durch den die Kontaktelemente 38, mit denen die Elektroden 36 versehen sind, nach oben herausragen. Die Vorrichtung 30 umfasst mindestens eine Aufnahmevorrichtung 32 zum Aufsetzen des Behältnisses 31. Die Aufnahmevorrichtung 32 ist mit einer Kühleinrichtung 33 versehen, die im vorliegenden Ausführungsbeispiel ein Peltier-Element ist. Die Kühleinrichtung ist in der hier dargestellten Ausführungsform mit einem Wärmeleiter 39 und einem Wärmeaustauscher 40 verbunden. Der Wärmetauscher 40 dient zur effizienten Abgabe der im Behältnis 31 entstandenen und durch die Kühleinrichtung 33 über den Wärmeleiter 39 transportierten Wärme an die Umwelt. Der Wärmetauscher 40 ist im dargestellten Beispiel ein mit Kühlrippen 41 versehener Körper. Zu Steigerung der Abgabe der im Prozess entstehenden Wärme kann die Vorrichtung 30 zusätzlich mit einem Ventilator versehen sein (nicht dargestellt). Die Kontaktmittel der Kontakteinrichtung (nicht dargestellt) üben bei der Kontaktierung der Kontaktelemente 38 der Elektroden 36 einen vertikal verlaufenden Druck auf das Behältnis 31 aus. Damit es durch diesen einseitigen Druck nicht zu einem Abkippen des Behältnisses 31 kommt, kann die erfindungsgemäße Vorrichtung 30 in vorteilhafter Ausgestaltung der Erfindung mindestens eine hier nicht dargestellte Anpresseinrichtung umfassen, welche das Behältnis 31 gleichmäßig auf die Aufnahmevorrichtung 32 und die Kühleinrichtung 33 presst. Die Anpresseinrichtung kann rahmenförmig ausgebildet und auf zumindest einen Teil des Randes des Behältnisses 31 aufsetzbar sein. Die Anpresseinrichtung kann auch Teil der Kontakteinrichtung sein. Insbesondere dient die Anpresseinrichtung dazu, die Luft aus dem Spalt 35 zwischen dem Behältnis 31 und der Aufnahmevorrichtung 32 zu verdrängen, um die Kontakt zwischen den Elektroden des Behältnisses 31 und der Kühleinrichtung 33 zu intensivieren. Auf diese Weise kann eine effektive Kühlung des Behältnisses 31 und der Elektroden sichergestellt werden.

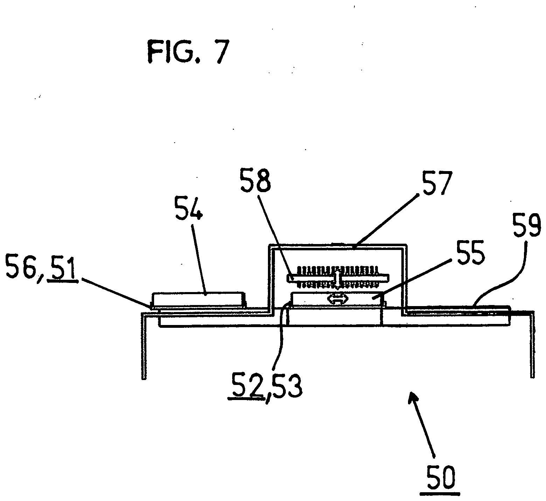

Figur 7 zeigt eine schematische Seitenansicht einer weiteren Ausführungsform einer erfindungsgemäßen Vorrichtung 50 mit linearer Bewegung der Aufnahmevorrichtungen. Die Vorrichtung 50 umfasst zwei Aufnahmevorrichtungen 51, 52, auf denen sich jeweils Behältnisse 54, 55 befinden. Die Aufnahmevorrichtung 52 befindet sich in dieser Darstellung innerhalb eines Gehäuses 57 auf der mittleren Position 53 und ist unterhalb einer Kontakteinrichtung 58 angeordnet. Mittels der Kontakteinrichtung 58 können die hier nicht dargestellten Elektroden des Behältnisses 55, vorzugsweise durch eine vertikale Bewegung der Kontakteinrichtung 58, elektrisch kontaktiert werden, um die Reaktionsräume des Behältnisses 55 mit einem oder mehreren Spannungsimpulsen zu beaufschlagen. Die Aufnahmevorrichtung 51 ist außerhalb des Gehäuses auf der linken Position 56 angeordnet, wobei diese Position 56 der Vor- und/oder Nachbereitung des Behältnisses 54 dient. Darüber hinaus befindet sich auf der gegenüberliegenden Seite des Gehäuses 57 eine weitere Position 59, welche Platz für die Kontakteinrichtung 55 bietet und der Vor- und/oder Nachbereitung des Behältnisses 55 dient. In dieser beispielhaften Ausführungsform der Erfindung können die Positionen der Behältnisse durch gekoppelte oder entkoppelte lineare Bewegungen gegeneinander ausgetauscht werden. Der Austausch der Behältnispositionen kann dabei entweder durch ein Bewegen der Behältnisse selbst oder ein Bewegen der Aufnahmevorrichtungen erreicht werden. Die erfindungsgemäße Vorrichtung 50 umfasst also zwei Aufnahmevorrichtungen 51, 52, die jeweils zwei Positionen 53 oder 56 bzw. 53 oder 59 einnehmen können (insgesamt sind drei Positionen 53, 56, 59 vorgesehen). Die Aufnahmevorrichtung 51 kann dabei zwischen der linken Position 56 und der mittleren Position 53 linear bewegt werden, während die Aufnahmevorrichtung 52 zwischen der mittleren Position 53 und der rechten Position 59 bewegt werden kann. Durch diesen Aufbau wird ermöglicht, immer das Behältnis in das Gehäuse 57 zu transportieren (Position 53), das als nächstes der elektrischen Behandlung unterzogen werden soll. Das andere Behältnis ist dann während der elektrischen Behandlung außerhalb des Gehäuses 57 für die Vor- und/oder Nachbereitung zugänglich (Positionen 56 und 59).

### Bezugszeichenliste:

- 1: Vorrichtung
- 2: Gehäuse
- 3: Aufnahmevorrichtung
- 4: Behältnis
- 5: Platte
- 6: Drehteller
- 7: Aufnahmevorrichtung
- 8: Kontakteinrichtung
- 9: Bügel
- 10: Bügel
- 11: Schiene
- 12: Schiene
- 13: Verteileinrichtung
- 14: Abdeckplatte
- 15: Behältnis
- 16: Achse
- 17: Kontaktmittel
- 20: Vorrichtung
- 21: Aufnahmevorrichtung
- 22: Behältnis
- 23: Verteileinrichtung
- 24: Kontaktmittel
- 25: Kontakteinrichtung
- 26: Kontaktelement
- 30: Vorrichtung
- 31: Behältnis
- 32: Aufnahmevorrichtung
- 33: Kühleinrichtung
- 34: Pfeil
- 35: Spalt
- 36: Elektrode
- 37: Deckel
- 38: Kontaktelement
- 39: Wärmeleiter
- 40: Wärmetauscher
- 41: Kühlrippen
- 50: Vorrichtung
- 51: Aufnahmevorrichtung
- 52: Aufnahmevorrichtung
- 53: Position
- 54: Behältnis
- 55: Behältnis
- 56: Position
- 57: Gehäuse
- 58: Kontakteinrichtung
- 59: Position

## Patentansprüche

1. Verfahren zur Beaufschlagung von mindestens zwei mit Elektroden versehenen Behältnissen (4, 15) mit mindestens einem Spannungsimpuls, bei dem mindestens ein Behältnis (4, 15) innerhalb eines Gehäuses (2) mit mindestens einem Spannungsimpuls beaufschlagt wird, während mindestens ein anderes Behältnis (4, 15) außerhalb des Gehäuses (2) einer Vor- oder Nachbereitung unterzogen wird, umfassend die folgenden Schritte:
a) Vorbereiten mindestens eines Behältnisses (4) außerhalb des Gehäuses (2) und Einbringen dieses Behältnisses (4) in das Gehäuse (2);
b) Beaufschlagen dieses Behältnisses (4) mit mindestens einem Spannungsimpuls innerhalb des Gehäuses (2) und gleichzeitig Vorbereiten mindestens eines weiteren Behältnisses (15) außerhalb des Gehäuses (2);
c) Gegenseitiges Tauschen der jeweiligen Positionen der Behältnisse (4, 15), wobei das bereits mit mindestens einem Spannungsimpuls beaufschlagte Behältnis (4) aus dem Gehäuse (2) heraus und das weitere Behältnis (15) in das Gehäuse hinein bewegt wird;
d) Beaufschlagen des weiteren Behältnisses (15) mit mindestens einem Spannungsimpuls innerhalb des Gehäuses (2) und gleichzeitig Nachbereiten des zuvor mit mindestens einem Spannungsimpuls beaufschlagten Behältnisses (4) sowie Vorbereiten mindestens eines nächsten Behältnisses außerhalb des Gehäuses (2).

2. Verfahren nach Anspruch 1, **dadurch gekennzeichnet, dass** die Behältnisse (4, 15) während der Schritte b) und d) jeweils mit mehreren Spannungsimpulsen beaufschlagt werden.

3. Verfahren nach Anspruch 1 oder 2, **dadurch gekennzeichnet, dass** die Behältnisse (4, 15) jeweils mindestens zwei mit jeweils mindestens einer Elektrode versehene Reaktionsräume umfassen und dass die Elektroden verschiedener Reaktionsräume sequenziell elektrisch kontaktiert werden.

4. Verfahren nach Anspruch 3, **dadurch gekennzeichnet, dass** eine Kontakteinrichtung (8) zur elektrischen Kontaktierung der Elektroden annähernd parallel zu mindestens einer Ebene der Kontaktierung bewegt und dann der elektrische Kontakt zu den Elektroden hergestellt wird.

5. Verfahren nach Anspruch 3 oder 4, **dadurch gekennzeichnet, dass** die Behältnisse (4, 15) mehrere Reaktionsräume mit jeweils mindestens einer Elektrode aufweist, wobei einzelne Gruppen von Reaktionsräumen festgelegt werden, und dass die Elektroden der Reaktionsräume einer Gruppe gleichzeitig und die Elektroden der Reaktionsräume verschiedener Gruppen sequenziell kontaktiert werden.

6. Verfahren nach einem der Ansprüche 1 bis 5, **dadurch gekennzeichnet, dass** mindestens ein Behältnis (4, 15) durch eine Kühleinrichtung gekühlt wird und dass das Behältnis (4, 15) annähernd gleichmäßig auf die Kühleinrichtung aufgepresst wird.

7. Verfahren nach Anspruch 6, **dadurch gekennzeichnet, dass** der Druck, mit dem das Behältnis (4, 15) auf die Kühleinrichtung aufgepresst wird, zumindest teilweise auf die Elektroden und/oder die Ränder des Behältnisses ausgeübt wird.

8. Vorrichtung (1, 20, 30) zur elektrischen Kontaktierung mindestens eines mit Elektroden versehenen Behältnisses (4, 15, 22, 31), mit mindestens zwei Aufnahmevorrichtungen (3, 7, 32) zum Aufsetzen mindestens eines Behältnisses (4, 15, 22, 31), mindestens einer Kontakteinrichtung (8, 25) zur Kontaktierung der Elektroden des Behältnisses (4, 15, 22, 31) und mit einem Gehäuse (2), in dem die Kontakteinrichtung (8, 25) angeordnet ist, wobei sich eine Aufnahmevorrichtung (3) an oder innerhalb der Kontakteinrichtung (8) befindet und wobei beide Aufnahmevorrichtungen (3, 7) und/oder die Kontakteinrichtung (8) derart bewegbar sind, dass sich die andere Aufnahmevorrichtung (7) nach Abschluss der Bewegung an oder innerhalb der Kontakteinrichtung (8) befindet, **dadurch gekennzeichnet, dass** eine Aufnahmevorrichtung (3, 7) innerhalb des Gehäuses (2) und die andere Aufnahmevorrichtung (3, 7) außerhalb des Gehäuses (2) angeordnet ist.

9. Vorrichtung nach Anspruch 8, **dadurch gekennzeichnet, dass** die Aufnahmevorrichtungen (3, 7) auf einer beweglichen Platte (5) angeordnet sind.

10. Vorrichtung nach Anspruch 8 oder 9, **dadurch gekennzeichnet, dass** mindestens ein Behältnis (22) mindestens zwei mit jeweils mindestens einer Elektrode versehene Reaktionsräume umfasst, wobei mindestens eine Kontakteinrichtung (25) vorgesehen ist, welche mindestens eine Kontakteinheit umfasst, die Kontaktmittel (24) zur Kontaktierung der Elektroden aufweist, wobei die Anzahl der Kontakteinheiten geringer als die Anzahl der Reaktionsräume ist und eine Kontakteinheit Kontaktmittel (24) zur Kontaktierung der Elektroden mindestens zweier Reaktionsräume aufweist.

11. Vorrichtung nach Anspruch 10, **dadurch gekennzeichnet, dass** die Kontakteinrichtung (25) trägerartig, vorzugsweise brücken- und/oder schlittenartig, ausgebildet und im Wesentlichen annähernd parallel zur Ebene der Kontaktierung bewegbar ist.

12. Vorrichtung nach Anspruch 10 oder 11, **dadurch gekennzeichnet, dass** zumindest ein Teil der Kontakteinrichtung (25) im Wesentlichen annähernd senkrecht zur Ebene der Kontaktierung bewegbar ist.

13. Vorrichtung nach einem der Ansprüche 8 bis 12, **dadurch gekennzeichnet, dass** die Aufnahmevorrichtung (32) mit einer Kühleinrichtung (33) versehen ist.

14. Vorrichtung nach Anspruch 13, **dadurch gekennzeichnet, dass** die Kühleinrichtung (33) mindestens ein Peltier-Element umfasst.

15. Vorrichtung nach Anspruch 13 oder 14, **dadurch gekennzeichnet, dass** mindestens eine Anpresseinrichtung vorgesehen ist, welche das Behältnis (31) auf die Aufnahmevorrichtung (32) und/oder die Kühleinrichtung (33) presst.

## Claims

1. Method for the application of at least one voltage pulse to at least two containers (4, 15) fitted with electrodes, by means of which at least one voltage pulse is applied to at least one container (4, 15) within a housing (2), while at least one other container (4, 15) is subjected to a preparation or a post-processing outside the housing (2), **comprising** the following steps:
a) Preparation of at least one container (4) outside the housing (2) and introducing of this container (4) into the housing (2);
b) Application of at least one voltage impulse to this container (4) within the housing (2) and at the same time preparation of at least one further container (15) outside the housing (2);
c) Mutual exchange of the respective positions of the containers (4, 15), wherein the container (4) to which a voltage pulse has already been applied is moved out of the housing (2) and the further container (15) is moved into the housing (2);
d) Application of at least one voltage pulse to the further container (15) within the housing (2) and at the same time post-processing of the container (4) to which at least one voltage pulse has been applied as well as preparation of at least one next container outside the housing (2).

2. Method according to claim 1, **characterised in that** a plurality of voltage pulses are applied to each container (4, 15) during steps b) and d).

3. Method according to claim 1 or 2, **characterised in that** each container (4, 15) comprises at least two reaction chamber fitted in each case with at least one electrode, and **in that** electrical contact is made sequentially with the electrodes of different reaction chambers.

4. Method according to claim 3, **characterised in that** a contact appliance (8) for making electrical contact with the electrodes is moved approximately parallel to at least one plane of contact and electrical contact is then made with the electrodes.

5. Method according to claim 3 or 4, **characterised in that** the containers (4, 15) have a plurality of reaction chambers with in each case at least one electrode, wherein individual groups of reaction chambers are determined, and **in that** contact is made with the electrodes of the reaction chambers of one group simultaneously and with the electrodes of the reaction chambers of various groups sequentially.

6. Method according to any of claims 1 to 5, **characterised in that** the container (4, 15) is cooled by a cooling appliance, and **in that** the container (4, 15) is pressed approximately evenly onto the cooling appliance.

7. Method according to claim 6, **characterised in that** the pressure, with which the container (4, 15) is pressed onto the cooling appliance, is exerted at least partly onto the electrodes and/or the edges of the container.

8. Device (1, 20, 30) for making electrical contact with at least one container (4, 15, 22, 31) fitted with electrodes, with at least two receptacles (3, 7, 32) upon which at least one container (4, 15, 22, 31) can be set, at least one contact appliance (8, 25) for contacting the electrodes of the container (4, 15, 22, 31), and with a housing (2) within which the contact appliance (8, 25) is disposed, wherein one receptacle (3) is located at or inside the contact appliance (8), and wherein both receptacles (3, 7) and/or the contact appliance (8) can be moved such that after completion of the movement the other receptacle (7) is located at or inside the contact appliance (8), **characterised in that** one receptacle (3, 7) is disposed inside the housing (2) and the other receptacle (3, 7) is disposed outside the housing (2).

9. Device according to claim 8, **characterised in that** the receptacles (3, 7) are arranged on a moveable plate (5).

10. Device according to claim 8 or 9, **characterised in that** at least one container (22) comprises at least two reaction chambers fitted in each case with at least one electrode, wherein at least one contact appliance (25) is provided, which comprises at least one contact unit having contact means (24) for contacting the electrodes, wherein the number of contact units is less than the number of reaction chambers and one contact unit has contact means (24) for contacting the electrodes of at least two reaction chambers.

11. Device according to Claim 10, **characterised in that** the contact appliance (25) is designed as a type of support, preferably as a type of bridge and/or slide, and can essentially be moved approximately parallel to the plane of contact.

12. Device according to claim 10 or 11, **characterised in that** at least one part of the contact appliance (25) can essentially be moved approximately perpendicular to the plane of contact.

13. Device (30) according to any of claims 8 to 12, **characterised in that** the receptacle (32) is fitted with a cooling appliance (33).

14. Device according to claim 13, **characterised in that** the cooling appliance (33) comprises at least one Peltier element.

15. Device according to claim 13 or 14, **characterised in that** at least one pressing device is provided, which presses the container (31) onto the receptacle (32) and/or the cooling appliance (33).

## Revendications

1. Procédé destiné à solliciter au moins deux récipients (4,15) dotés d'électrodes avec au moins une impulsion de tension pour lequel au moins un récipient (4,15) est sollicité à l'intérieur d'un boîtier (2) avec au moins une impulsion de tension, pendant qu'au moins un autre récipient (4,15) en dehors du boîtier (2) est soumis à une préparation préliminaire ou ultérieure, comprenant les étapes suivantes :
a) préparation d'au moins un récipient (4) en dehors du boîtier (2) et incorporation de ce récipient (4) dans le boîtier (2),
b) sollicitation de ce récipient (4) avec au moins une impulsion de tension à l'intérieur du boîtier (2) et simultanément préparation d'au moins un autre récipient (15) en dehors du boîtier (2),
c) échange réciproque des positions respectives des récipients (4,15), le récipient (4) déjà sollicité avec au moins une impulsion de tension étant déplacé hors du boîtier (2) et l'autre récipient (15) à l'intérieur du boîtier,
d) sollicitation de l'autre récipient (15) avec au moins une impulsion de tension à l'intérieur du boîtier (2) et simultanément préparation ultérieure du récipient (4) préalablement sollicité avec au moins une impulsion de tension ainsi que préparation d'au moins un récipient suivant en dehors du boîtier (2).

2. Procédé selon la revendication 1, **caractérisé en ce que** les récipients (4,15) sont sollicités respectivement avec plusieurs impulsions de tension pendant les étapes b) et d).

3. Procédé selon la revendication 1 ou 2, **caractérisé en ce que** les récipients (4,15) comprennent respectivement au moins deux espaces de réaction dotés d'au moins une électrode et **en ce que** les électrodes de différents espaces de réaction sont électriquement mises en contact de façon séquentielle.

4. Procédé selon la revendication 3, **caractérisé en ce qu'**un dispositif de contact (8) est déplacé pour un contact électrique des électrodes à peu près parallèlement à au moins un plan de contact et le contact électrique est établi ensuite avec les électrodes.

5. Procédé selon la revendication 3 ou 4, **caractérisé en ce que** les récipients (4,15) comportent plusieurs espaces de réaction avec respectivement au moins une électrode, des groupes individuels d'espaces de réaction étant définis et **en ce que** les électrodes des espaces de réaction d'un groupe sont simultanément mises en contact et les électrodes des espaces de réaction de différents groupes le sont de façon séquentielle.

6. Procédé selon l'une quelconque des revendications 1 à 5, **caractérisé en ce qu'**au moins un récipient (4,15) est refroidi par un dispositif de réfrigération et **en ce que** le récipient (4,15) est engagé par pression à peu près uniformément sur le dispositif de réfrigération.

7. Procédé selon la revendication 6, **caractérisé en ce que** la pression avec laquelle le récipient (4,15) est engagé par pression sur le dispositif de réfrigération est au moins exercée en partie sur les électrodes et/ou les bords du récipient.

8. Dispositif (1,20,30) destiné au contact électrique d'au moins un récipient (4,15,22,31) doté d'électrodes avec au moins deux dispositifs de réception (3,7,32) pour la mise en place d'au moins un récipient (4,15,22,31), d'au moins un dispositif de contact (8,25) pour le contact des électrodes du récipient (4,15,22,31) et avec un boîtier (2) dans lequel le dispositif de contact (8,25) est disposé, un dispositif de réception (3) se trouvant sur ou à l'intérieur du dispositif de contact (8) et les deux dispositifs de réception (3,7) et/ou le dispositif de contact (8) étant mobiles de telle sorte que l'autre dispositif de réception (7) se trouve sur ou à l'intérieur du dispositif de contact (8) après la fin du mouvement, **caractérisé en ce qu'**un dispositif de réception (3,7) est disposé à l'intérieur du boîtier (2) et l'autre dispositif de réception (3,7) en dehors du boîtier (2).

9. Dispositif selon la revendication 8, **caractérisé en ce que** les dispositifs de réception (3,7) sont disposés sur une plaque mobile (5).

10. Dispositif selon la revendication 8 ou 9, **caractérisé en ce qu'**au moins un récipient (22) comprend au moins deux espaces de réaction dotés respectivement d'au moins une électrode, au moins un dispositif de contact (25) étant prévu, lequel comprend au moins une unité de contact qui comporte les moyens de contact (24) pour la mise en contact des électrodes, le nombre des unités de contact étant inférieur au nombre des espaces de réaction et une unité de contact comportant des moyens de contact (24) pour la mise en contact des électrodes d'au moins deux espaces de réaction.

11. Dispositif selon la revendication 10, **caractérisé en ce que** le dispositif de contact (25) est configuré du type porteur, de préférence de type pont et/ou coulisseau et peut être mobile pour l'essentiel à peu près parallèlement au plan de la mise en contact.

12. Dispositif selon la revendication 10 ou 11, **caractérisé en ce qu'**au moins une partie du dispositif de contact (25) est mobile pour l'essentiel à peu près perpendiculairement au plan de la mise en contact.

13. Dispositif selon l'une quelconque des revendications 8 à 12, **caractérisé en ce que** le dispositif de réception (32) est doté d'un dispositif de réfrigération (33).

14. Dispositif selon la revendication 13, **caractérisé en ce que** le dispositif de réfrigération (33) comprend au moins un élément de Peltier.

15. Dispositif selon la revendication 13 ou 14, **caractérisé en ce qu'**au moins un dispositif de compression est prévu, lequel comprime le récipient (31) sur le dispositif de réception (32) et/ou le dispositif de réfrigération (33).
